# EUROPEAN PATENT APPLICATION

(11) **EP 4 732 779 A1**
(43) Date of publication of application: **29.04.2026**
(21) Application number: 24208966.2
(22) Date of filing: 25.10.2024
(51) Int. Cl.: A61B 7/00, A61B 5/087

(54) **AN ACOUSTIC SENSING SYSTEM**

(71) Applicant: Koninklijke Philips N.V., 5656 AG Eindhoven (NL)
(72) Inventor: DE GRAAF, Pascal, Eindhoven (NL); DE VRIES, Jan Johannes Gerardus, Eindhoven (NL)
(74) Representative: Philips Intellectual Property & Standards

(57) **Abstract**

An acoustic sensing system is provided for monitoring sounds including sounds from a subject's airway. An acoustic sensor generates an acoustic signal, and a pressure or flow sensor generates a pressure or flow signal relating to the subject's airway. The acoustic signal and the pressure or flow signal are processed to identify secretions. In one aspect, the acoustic signal is processed to filter out sounds associated with the secretions. In another aspect, for each identified secretion, a type is identified from a plurality of different secretion types.

## Description

### FIELD OF THE INVENTION

This disclosure relates to acoustic sensing, and in particular in the presence of acoustic noise comprising sounds generated within the airway of a subject, caused by mucus secretions.

### BACKGROUND OF THE INVENTION

Acoustic sensors are widely used in a variety of applications because of the unique combination of benefits: small size, low cost, low power, no line-of-sight requirement and good reliability.

A general problem of integrating acoustic sensors in devices is obtaining a high signal-to-noise ratio (SNR). In particular, the detected acoustic signal may be a combination of the signal of interest as well as sources of noise.

Airway sounds will for example be present in acoustic signal monitoring performed as part of sleep and respiratory care applications. Such applications include continuous positive airway pressure (CPAP) machines, spirometry systems, mechanical insufflator-exsufflator devices, breathing training devices etc.

It is for example known to use an acoustic sensor in a CPAP machine for device diagnostics. WO 2021/207796 discloses the use of acoustic sensing for many purposes such as mask identification and leak detection of a ventilator. It is also known to use acoustic sensing and/or pressure sensing for patient monitoring and diagnosis.

Especially in sleep and respiratory care devices, the sound signals are noisy and may also significantly vary between devices, users and over time. This poses challenges to signal processing, analysis and feature classification and therefore it may lead to false detection of device deterioration, wrong hardware identification or inaccurate patient monitoring. There is a need for solutions that improve the acoustic detection, and this can in turn improve the monitoring of devices, users and therapies.

### SUMMARY OF THE INVENTION

The invention is defined by the claims.

According to examples in accordance with this disclosure, there is provided an acoustic sensing system for monitoring sounds including sounds from a subject's airway, comprising:
an acoustic sensor for generating an acoustic signal;
a pressure or flow sensor for generating a pressure or flow signal relating to the subject's airway;
a signal processor, configured to:
   process the acoustic signal and the pressure or flow signal to identify secretions.

This disclosure relates to acoustic sensing that is used as part of a device for which sounds generated by the airway of a subject are detected.

The airway sounds may for example be generated by airway secretions. Airway secretions can have different effects based on their position in the airway. Secretions in the alveoli make audible sounds, whereas secretions in the upper airway can create low frequency pressure oscillations (5 - 20Hz). Generally, a combination of both is present, and the composition can change throughout the day. The detection and identification of such secretions would improve planning of mucus management in the form of medication, physiotherapy or mechanical removal.

This system identifies secretions based on both acoustic and pressure characteristics of those secretions. This provides a more robust detection approach than existing ways to identify secretions.

The signal processor may be configured to process the acoustic signal to filter out sounds associated with the secretions.

In this way, once identified, the acoustic signal is processed to filter out sounds that can be attributed to the secretions, thereby the acoustic signal has an improved signal to noise ratio. The system can be integrated into a device such as a ventilator. This may for example improve planning of mucus management in the form of medication, physiotherapy or mechanical removal.

The pressure or flow signal relating to the subject's airway is for example a pressure that is measured in a conduit that is used to deliver breathing gas to the patient's airway.

The signal processor for example comprises a transformer for transforming the acoustic signal in the time domain to a frequency domain acoustic signal in the frequency domain, and for transforming the pressure or flow signal in the time domain to a frequency domain pressure or flow signal in the frequency domain. Frequency domain characteristics of the pressure and acoustic signals may be used to identify secretions.

The signal processor is for example configured to apply a classifier, wherein the classifier is configured to provide an output representative of a classification based on a frequency spectrum of the frequency domain acoustic signal and based on a frequency spectrum of the frequency domain pressure or flow signal, and the signal processor is configured to identify the secretions based on the classification. Thus, the presence of certain frequency components is indicative of different secretion types. Frequency thresholds may be applied that require selected frequencies to be present with a corresponding amplitude threshold level.

In a first signal processing approach, the signal processor is configured to concatenate the frequency spectrum of the frequency domain acoustic signal and the frequency spectrum of the frequency domain pressure or flow signal. This provides a combined frequency spectrum of both pressure and acoustic signal characteristics.

In a second signal processing approach, the signal processor is configured to concatenate feature vectors of the frequency spectrum of the frequency domain acoustic signal and feature vectors of the frequency spectrum of the frequency domain pressure or flow signal. In this way, feature vectors are defined separately for the acoustic signal and the pressure or flow signal, and they are combined.

In a third signal processing approach, the signal processor is configured to average the frequency spectrum of the frequency domain acoustic signal and the frequency spectrum of the frequency domain pressure or flow signal.

In a fourth signal processing approach, the classifier that is applied comprises a first classifier and a second classifier and the signal processor is configured to apply the first classifier when processing the frequency domain acoustic signal and to apply the second classifier when processing the frequency domain pressure or flow signal, and combine the outputs of the first and second classifiers.

The signal processor may for example sum the acoustic signal and pressure or flow signal before performing subsequent analysis.

Thus, there are various ways to combine the acoustic and pressure or flow signals, at different stages of the signal processing pipeline.

In all cases, the signal processor may be configured to:
apply an algorithm that identifies the acoustic signal components and the pressure or flow signal components that resulted in an identified secretion; and
process the acoustic signal to filter out the acoustic signal components.

In this way, the detected pressure and acoustic signals result in identification of a secretion. The components of the acoustic signal that resulted in the identification are identified by the algorithm so that those specific components can be filtered out, for example using tunable digital filters, from the acoustic signal.

The signal processor may be configured to identify a type from a plurality of different secretion types. A secretion type may indicate a mucus location along the airway for example. Thus, different types correspond to different mucus locations. This may be of interest for diagnostic and treatment purposes. Secretions at different locations along the airway will result in different characteristic pressure and acoustic signals. For each identified secretion, an indication of mucus distribution and severity may also be derived from the pressure and sound characteristics.

According to another aspect of this disclosure, there is provided a ventilator comprising:
a pressure source for delivering air under pressure to a subject's mouth and/or nose; and
the acoustic sensing system as defined above.

The ventilator may further comprise a diagnostic system for performing system diagnostics using the processed acoustic signal. Patient diagnostics may also be performed using the pressure or flow signal.

According to another aspect of this disclosure, there is provided a signal processing method comprising:
receiving an acoustic signal capturing sounds including sounds from a subject's airway;
receiving a pressure or flow signal relating to the subject's airway;
processing the acoustic signal and the pressure or flow signal to identify secretions.

In one aspect, the method may comprise processing the acoustic signal to filter out sounds associated with the secretions.

In another aspect, the method may comprise, for each identified secretion, identifying a type from a plurality of different secretion types.

This disclosure also provides a computer program comprising computer program code which is adapted, when said program is run on a computer, to implement the methods defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an acoustic sensing system;
Fig. 2 shows a ventilator using the acoustic sensing system;
Fig. 3 shows an acoustic signal processing method; and
Fig. 4 shows the general architecture of the processing unit uses in the system.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

This disclosure relates to an acoustic sensing system for monitoring sounds including sounds from a subject's airway. An acoustic sensor generates an acoustic signal, and a pressure or flow sensor generates a pressure or flow signal relating to the subject's airway. The acoustic signal and the pressure or flow signal are processed to identify secretions. In one aspect, the acoustic signal is processed to filter out sounds associated with the secretions. In another aspect, for each identified secretion, a type is identified from a plurality of different secretion types.

In this way, instead of processing only data from an acoustic sensor, the data collected by a pressure or flow sensor is also used. Using one ore more classifiers, the sound and pressure/flow signatures of secretions can be identified to provide information about the secretions, and the sound signature can be filtered out of the audio signal to improve the signal-to-noise ratio thereby to improve further acoustic analysis.

Airway secretions can have different effects based on their position in the airway. Secretions in the alveoli can produce characteristics sounds, such as a wheeze of a COPD patient, with a frequency above 400 Hz. However, the sound can vary widely over a five-octave range. Secretions in the upper airway can create low frequency pressure oscillations (e.g. in the range 5Hz to 20Hz) that are visible in the ventilator waveforms.

For example, a flow-volume curve of a patient requiring mucus clearance due to mucus accumulation has a characteristic noisy sawtooth patten in the inspiratory phase, as explained in US 2017/0303821.

In order to detect the presence of such frequencies, raw waveform data coming from the sensors is for example transformed into the frequency spectrum, e.g., by applying a (Fast) Fourier Transform.

Generally, secretions present a combination of both acoustic characteristics and pressure characteristics. Thus, by combining data from both a pressure or flow sensor and an acoustic sensor, a more complete picture can be obtained about the secretion distribution throughout the lungs and severity than is possible with only one modality. Separate pressure and flow sensing may also be employed in addition to acoustic sensing.

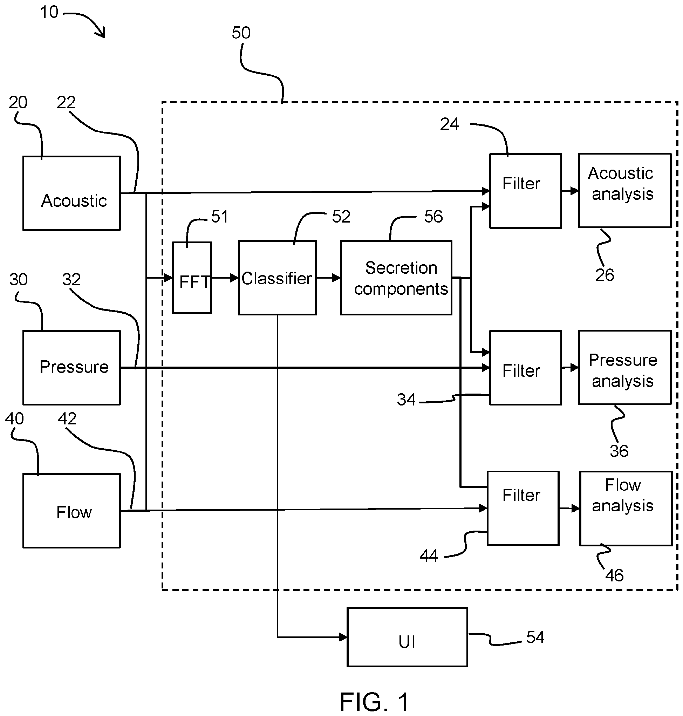

Fig. 1 shows an acoustic sensing system 10 for monitoring sounds including sounds from a subject's airway.

The system comprises an acoustic sensor 20 for generating an acoustic signal 22 and a pressure sensor 30 for generating a pressure signal 32 relating to the subject's airway. The pressure sensor 30 for example measures a pressure at a location that is fluidly coupled to the patient's airway, for example the pressure at a conduit leading to a patient interface (mask). Fig. 1 also shows a flow sensor 40 for generating a flow signal 40.

The system may comprise an acoustic sensor and a pressure sensor, or an acoustic sensor and a flow sensor, or all three sensor types.

In the following description, it will be assumed that the system is configured as an acoustic sensor and a pressure sensor. However, all mentions of pressure sensing may be replaced with flow sensing in an alternative implementation.

The acoustic sensing system is for example integrated into a host system, such as a sleep monitoring or sleep care system or a breathing monitoring or breathing assistance system. For example, the host system is a ventilator. The acoustic sensor may be any form of microphone, and it may be located at any position where it can receive sounds generated by the host system hardware as well as sounds generated within the airway of the subject using the system. The acoustic sensor may be located within the host system. For example, in the case of a ventilator it may be located within the main ventilator housing in which the pressure source is located, or it may be located at the mask to be coupled to the ventilator housing. Similarly, the pressure sensor may be located within the host system. For example, in the case of a ventilator the pressure sensor may be located within the main ventilator housing in which the pressure source is located, or it may be located at the mask to be coupled to the ventilator housing, or it may be located along a delivery conduit between the mask and the main ventilator housing.

A signal processor 50 processes the acoustic signal 22 and the pressure signal 32 (and/or the flow signal 42) to identify secretions. For this purpose, the signal processor 50 comprises a transformer 51. The transformer 51 is adapted to transform the acoustic signal in the time domain to a frequency domain acoustic signal in the frequency domain. The transformer 51 is adapted to transform the pressure signal from the time domain to a frequency domain pressure signal in the frequency domain. The frequency domain acoustic signal and the frequency domain pressure signal are further referred to as frequency domain signals. The secretions are detected using a classifier 52 that operates on the frequency domain signals. The results of the classification are output using a user interface 54 such as a display, or a wireless communication system for transmitting a signal to a remote display device.

The classifier 52 also identifies the secretion components 56 of the acoustic and pressure signals that resulted in the particular classification. This then enables the acoustic signal to be processed using filter 24 to filter out sounds associated with the secretions. Similarly, the pressure signal may be processed using filter 34 to filter out pressure fluctuations associated with the secretions. If a flow signal is used, it may be processed by filer 44 to filter out flow fluctuations associated with the secretions.

In this way, when the acoustic sensing system identifies the presence of a certain type of secretion, the measured acoustic and pressure frequencies that lead to this classification may be filtered out of the audio and pressure signals, thereby improving the signal to noise ratio for subsequent acoustic analysis 26 and pressure analysis 36. If a flow signal is used, flow analysis 46 of the filtered signal may be performed. This can be achieved by applying digital filters such as band-stop filters and/or band-pass filters.

This example of the system identifies secretions based on at least acoustic and pressure characteristics of those secretions. Once identified, the acoustic signal may be processed to filter out sounds that can be attributed to the secretions, thereby creating an acoustic signal with improved signal to noise ratio. The secretion detection is more robust by classification using at least acoustic and pressure signals.

The operation of the system will be described in more detail, again using an example based on acoustic and pressure sensing only. The processing is performed on the combination of the acoustic signal and pressure signal. For this purpose, data fusion of the acoustic signal and the pressure signal is performed.

There are for example three levels of data fusion that may be used to combine the information present in both sensor signals.

A first data fusion approach is based on signal (or sensor) level fusion, so that a single data stream is generated at the sensor side. This can for example be achieved by summing the raw acoustic and pressure waveforms, or by averaging the frequency spectra of the two waveforms. This frequency averaging combines the frequency spectra of the two signals by averaging the components in corresponding frequency bins of two frequency histograms representing the frequency spectra. This approach means the subsequent processing steps can be simplified in that a single data stream is processed. However, information about the source of specific frequencies (pressure or acoustic) is lost.

A second data fusion approach is feature level fusion. This may be achieved by concatenating the frequency spectra or by concatenating feature vectors obtained from the processing of the frequency spectra. This has the advantage that the processed information can be traced back to the individual sensors, and it allows cross correlation analysis between the information from the two sensors.

The feature vectors for example describe characteristics of the frequency histograms, such as the power level within a given frequency range, or a ratio between the power levels of two frequency ranges, or a frequency with the highest power, or a number of number of peaks in the frequency spectrum, etc.

By using an algorithm that is able to identify the acoustic signal components and the pressure signal components that resulted in an identified secretion, those specific components can be filtered out in the filtering stage, for example using tunable digital filters.

A third data fusion approach is decision level fusion. In this case, two separate processing pipelines may be used, including classifiers, in parallel. Only the outcome of the classifiers is then combined. This gives a simple implementation, but it loses information present in cross correlations between sensor signals.

The classifier 52 is applied to the fused data, which may take the various forms as outlined above. One implementation uses so-called open-box classification techniques, such as decision trees, random forests, and learning vector quantization, which enable inspection of the learned rules/mechanisms and enable discovery of not-yet-known phenotypes. The use of such techniques enables easier extraction of which frequencies were involved in each particular classification, as explained above. Other techniques may instead require hard-coding the frequencies involved when classifying to each of the classes (e.g., phenotypes).

In a simplest form, the classifier 52 uses frequency thresholds as decision boundaries. The presence of certain frequency components is indicative of different secretion types. The frequency thresholds for example require selected frequencies to be present with a minimum amplitude level. In a simplest form, a single given frequency range may be compared against a single threshold. However, multiple frequency ranges may be compared against respective thresholds, that may be the same or different.

To improve robustness of the system, the classifier 52 may use a labelled dataset. The labelled dataset comprises multiple acoustic signals and pressure signals with annotated disease states and/or annotated secretion distribution corresponding to the multiple acoustic signals and pressure signals. A learning procedure may be used to optimize the classifier 52 to provide the classification given the data from the acoustic sensor and pressure sensor. In this way, the classifier 52 can optimize the preconfigured frequency thresholds, but also learn additional rules that exclude or include cases where additional frequencies are present.

For example, machine learning may be used to provide a self-learning analysis of the pressure and acoustic signals, by training a machine learning algorithm using ground truth data from historical patient data.

The acoustic sensing system is able to identify various phenotypes of mucus distribution and severity independent of other variables, which can be communicated to the user or caregiver to help with mucus management planning. This is achieved using user interface 54. This may comprise a display on the host system itself, or it may comprise a communication system for providing data to a data collection system. For example, the results could be expressed in a visual form of the lungs with color gradients for secretion presence, or presented as a list of lung sections (tracheal, bronchial, vesicular) with corresponding presence (expressed in low/med/high, or approximate layer thickness, depending on the accuracy).

It is noted that it is known that a pressure signal and/or a flow signal of a ventilator may be analyzed to derive mucus characteristics, such as mucus thickness, mucus viscosity and distribution of mucus in the airway. This is for example disclosed in WO 2022/200139. The approach disclosed in WO 2022/200139 may be applied to the sensed pressure in the acoustic sensing system described above. The use of acoustic sensing in an addition to pressure sensing (and/or flow sensing) provides additional robustness to the classification of mucus characteristics.

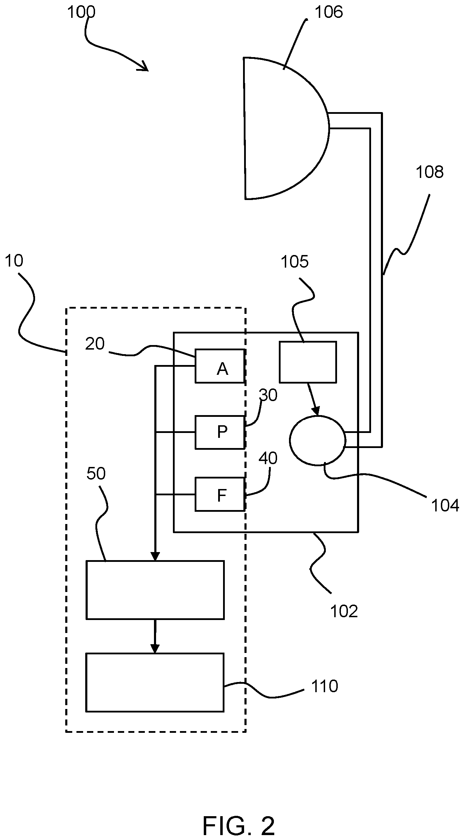

Fig. 2 shows a ventilator 100 comprising a main housing 102 having a breathing gas delivery pump 104, pump controller 105, and a patient interface (e.g., a mask) 106 coupled to the main housing 102 by a supply conduit 108.

The acoustic sensing system 10 described above monitors the pressure and flow rate of the breathing gas delivered to the user by the patient interface 106. For example, the sensors are located in the main housing 102, or are remote from the main housing 102, such as in the patient interface 106 or in the supply conduit 108. In addition to the signal processor 50, there is a diagnostic system 110 for performing system diagnostics using the processed acoustic signal. Patient diagnostics may also be performed using the pressure signal.

System diagnostics for example involve identifying leakage sounds, identifying water in hoses, detecting device failures and/or monitoring pump function. Examples of diagnoses which may use an acoustic signal are disclosed in WO 2021/207796.

Pressure monitoring is for example used in a CPAP machine to maintain a target pressure, so that a constant, steady flow of air is delivered at a specific pressure to keep the airway open in individuals with obstructive sleep apnea (OSA). If the pressure drops (due to leaks, for example), the machine will adjust airflow to maintain the target pressure.
A pressure sensor may be used to detect changes in a user's breathing patterns throughout the night. The pressure can then be adjusted automatically based on the sensed airway resistance, increasing the pressure when there are signs of an obstruction and lowering the pressure when normal breathing resumes. Pressure sensing may be used to detect air leaks around the patient interface.

The improved pressure signal obtained by filtering as explained above improves the robustness of the pressure monitoring.

Patient diagnoses for example involves monitoring breathing patterns to detect issues such as apneas or hypopneas. The detection of inhalation and exhalation is used by Bilevel Positive Airway Pressure, BiPAP, devices to provide two levels of pressure (one for inhalation and one for exhalation).

The improved signal to noise ratio of the acoustic and/or pressure sensing signal again improves the robustness of these diagnostic functions.

The information about secretion type and severity may be used to improve planning of mucus management in the form of medication, physiotherapy or mechanical removal. For example, a secretion type may be identified from a plurality of different secretion types (e.g. at different locations within the airway).

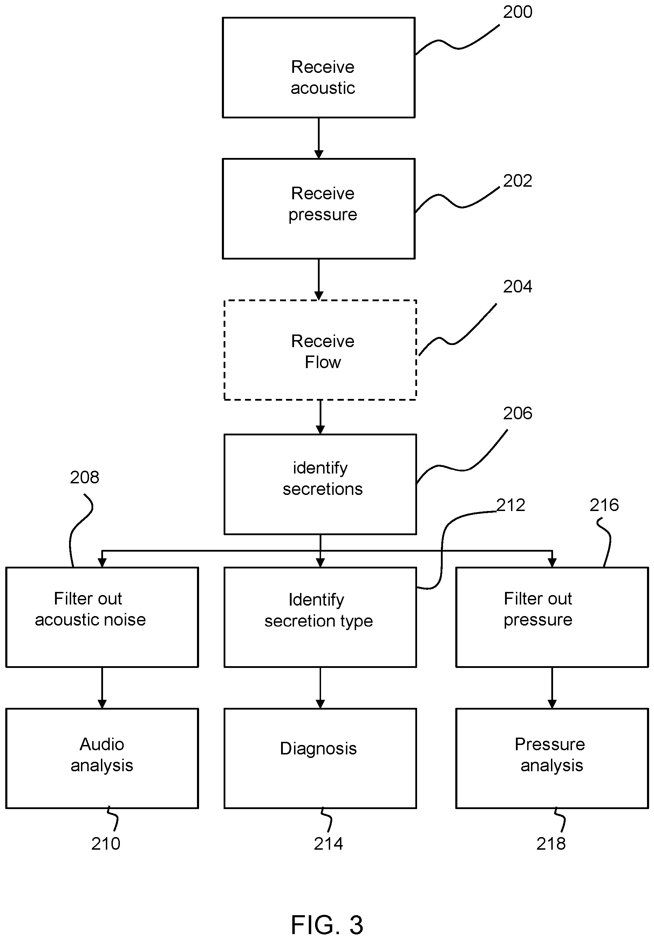

Fig. 3 shows a signal processing method.

In step 200, an acoustic signal is received capturing sounds including sounds from a subject's airway.

In step 202, a pressure signal is received relating to the subject's airway.

In step 204, optionally, a flow signal is received.

In step 206, the signals are processed to identify secretions.

The acoustic signal is processed in step 208 to filter out sounds associated with the secretions so that improved audio analysis is possible in step 210. This is one aspect of this disclosure.

A secretion type is identified in step 212, and optionally also a secretion severity. This is another aspect of this disclosure. In this way, a diagnosis or treatment management decision can be made in step 214. In an embodiment, the steps 200, 202, 206, and 212 are performed by the acoustic sensing system 10, whereas step 214 is performed by a medical practitioner based on the secretion type identified in step 212.

The pressure signal is processed in step 216 to filter out pressures associated with the secretions so that improved pressure analysis is possible in step 218.

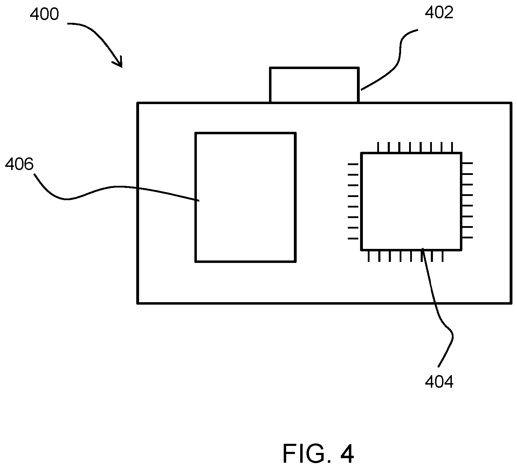

The system above uses a signal processor 50 for processing the sensor signals. Fig. 4 shows a signal processor 400 comprising an I/O interface 402, a controller 404 and a memory 406. The I/O interface 402, the controller 404 and the memory 406 may be attached to a printed circuit board (PCB). The control unit 404 may receive and send signals or data via the I/O interface 402. The I/O interface 402 may be a wireless interface or a connector. The signal processor 400 may store the data or signals received by the control unit 404 in the memory 406. The memory 406 may contain additional data or executable computer program products, for example in terms of a computer-implemented method, that may be retrieved, processed or executed by the controller 404. Data or signals resulting from the processing of data or signals or from the execution of a computer program product may be stored to the memory 406 or sent to the I/O interface 402 by the controller 404. The control unit 404 may be a standalone control unit or it may represent a network of multiple control units distributed within the system.

This disclosure is of particular interest to ventilators, for example for sleep monitoring, sleep treatment, and/or respiratory care. It may be used for monitoring and diagnosis, for example for monitoring patients with cystic fibrosis.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. An acoustic sensing system for monitoring sounds including sounds from a subject's airway, comprising:
an acoustic sensor (20) for generating an acoustic signal;
a pressure or flow sensor (30) for generating a pressure or flow signal relating to the subject's airway;
a signal processor (50), configured to:
process the acoustic signal and the pressure or flow signal to identify secretions.

2. The system of claim 1 wherein the signal processor is further configured to:
process the acoustic signal to filter out sounds associated with the secretions.

3. The system of claim 1 or 2, wherein the signal processor (50) comprises a transformer (51) for transforming the acoustic signal in the time domain to a frequency domain acoustic signal in the frequency domain, and for transforming the pressure or flow signal in the time domain to a frequency domain pressure or flow signal in the frequency domain.

4. The system of claim 3, wherein the signal processor (50) is configured to apply a classifier,
wherein the classifier is configured to provide an output representative of a classification based on a frequency spectrum of the frequency domain acoustic signal and based on a frequency spectrum of the frequency domain pressure or flow signal,
wherein the signal processor (50) is configured to identify the secretions based on the classification.

5. The system of claim 3 or 4, wherein the signal processor is configured to:
concatenate the frequency spectrum of the frequency domain acoustic signal and the frequency spectrum of the frequency domain pressure or flow signal; or
concatenate feature vectors of the frequency spectrum of the frequency domain acoustic signal and feature vectors of the frequency spectrum of the frequency domain pressure or flow signal; or
average the frequency spectrum of the frequency domain acoustic signal and the frequency spectrum of the frequency domain pressure or flow signal.

6. The system of claim 3 or 4, wherein the classifier comprises a first classifier and a second classifier,
wherein the signal processor is configured to apply the first classifier when processing the frequency domain acoustic signal and to apply the second classifier when processing the frequency domain pressure or flow signal, and combine the outputs of the first and second classifiers.

7. The system of any one of claims 1 to 4, wherein the signal processor is configured to sum the acoustic signal and pressure or flow signal.

8. The system of any one of claims 1 to 7, wherein the signal processor is configured to:
apply an algorithm that identifies the acoustic signal components and the pressure or flow signal components that resulted in an identified secretion; and
process the acoustic signal to filter out the acoustic signal components.

9. The system of any one of claims 1 to 8, wherein the signal processor is further configured, for each identified secretion, to identify a type from a plurality of different secretion types.

10. The system of claim 9, wherein the signal processor (50) is configured, for each identified secretion, to provide an indication of mucus distribution and severity.

11. A ventilator (100) comprising:
a pressure source (104) for delivering air under pressure to a subject's mouth and/or nose; and
the acoustic sensing system (10) of any one of claims 1 to 10.

12. The ventilator of claim 11, further comprising a diagnostic system (110) for performing system diagnostics using the processed acoustic signal.

13. A signal processing method comprising:
(200) receiving an acoustic signal capturing sounds including sounds from a subject's airway;
(202) receiving a pressure or flow signal relating to the subject's airway;
(206) processing the acoustic signal and the pressure or flow signal to identify secretions.

14. The method of claim 13, further comprising:
(208) processing the acoustic signal to filter out sounds associated with the secretions; and/or
(212) for each identified secretion, identifying a type from a plurality of different secretion types.

15. A computer program comprising computer program code which is adapted, when said program is run on a computer, to implement the method of claim 13 or 14.
